# EUROPÄISCHE PATENTSCHRIFT

(11) **EP 2 997 958 B1**
(45) Veröffentlichungstag und Bekanntmachung des Hinweises auf die Patenterteilung: **10.03.2021**
(21) Anmeldenummer: 15185577.2
(22) Anmeldetag: 17.09.2015
(51) Int. Cl.: A61K 8/06, A61K 8/41, A61K 8/73, A61K 8/81, A61Q 5/12

(54) **EMULSION ENTHALTEND FLÜSSIGE ESTERQUATS UND POLYMERVERDICKER**
EMULSION COMPRISING LIQUID ESTER QUATS AND POLYMER THICKENER
ÉMULSION CONTENANT DES ESTERQUATS LIQUIDES ET POLYMÈRES ÉPAISSISSANTS

(30) Priorität: 22.09.2014 EP 14185779
(43) Veröffentlichungstag der Anmeldung: 23.03.2016
(73) Patentinhaber: Evonik Operations GmbH, 45128 Essen (DE)
(72) Erfinder: Schwab, Peter, 45133 Essen (DE); Westerholt, Ursula, 45138 Essen (DE); Kortemeier, Uta, 45128 Essen (DE); Kleinen, Jochen, 52525 Heinsberg (DE); Hartung, Christian, 45133 Essen (DE); Köhle, Hans-Jürgen, 63533 Mainhausen (DE)
(74) Vertreter: Evonik Patent Association

(56) Entgegenhaltungen:
- EP-A2- 0 581 442
- EP-A2- 2 783 677
- DE-A1-102012 205 088
- US-A1- 2004 005 286
- US-A1- 2006 140 899
- US-A1- 2013 225 470
- US-B1- 6 376 455
- US-B1- 7 578 857

## Beschreibung

### Gebiet der Erfindung

Gegenstand der Erfindung sind Formulierungen enthaltend flüssige Esterquats und Polymerverdicker sowie ihre Verwendung.

### Stand der Technik

Haarkonditionierformulierungen auf Basis flüssiger Esterquats und/oder Imidazoliniumsalze zeichnen sich durch hervorragende Konditioniereigenschaften und einfache und vielseitige Verwendbarkeit aus. Allerdings ist die Langzeitstabilität einfacher Formulierungen im Vergleich zu festen Esterquats und/oder Imidazoliniumsalze mangelhaft. Die Emulsionsstabilisierung gestaltet sich als schwierig.

Die US6376455 offenbart in Beispiel 7 eine Formulierung enthaltend Hydroxyethylcellulose und ein quaternisiertes Veresterungsprodukte von Methyl-Ethyl-Isopropanolamin. Nachstellungen ergeben, dass das eingesetzte Esterquat einen Schmelzpunkt von mindestens 85°C bei 1 bar aufweist.

Die EP2783677 offenbart kosmetische Formulierungen enthaltend Esterquats aufweisend einen Acylrest einer mindestens einfach ungesättigten Fettsäure mit einer Kettenlänge von 18 bis 24 Kohlenstoffatomen oder ein Acylrest der Isostearinsäure oder Rizinolsäure und Wasser.

Aufgabe der Erfindung war es, lagerstabile Formulierungen enthaltend Esterquats bereitzustellen.

### Beschreibung der Erfindung

Überraschenderweise wurde nun gefunden, dass Formulierungen enthaltend flüssige Esterquats durch Zugabe von Carbomeren oder anderen Polymeren langzeitstabiler gemacht werden können. Insbesondere im Falle der Carbomere ist dies überraschend, da diese normalerweise aufgrund der Wechselwirkung mit kationischen Tensiden zu einer Phasentrennungen führen und in Haarkonditionierformulierung somit bisher nicht eingesetzt werden können. Gegenstand der vorliegenden Erfindung sind daher Formulierungen enthaltend mindestens ein flüssiges Esterquat (Komponente A)) und mindestens einen Polymerverdicker (Komponente B)), wie in Anspruch 1 beschrieben. Beschrieben wird weiterhin die Verwendung der erfindungsgemäßen Formulierungen in kosmetischen sowie textilen Anwendungen.

Ein Vorteil der vorliegenden Erfindung ist es, dass die eingesetzten Esterquats bei Raumtemperatur flüssig sind und somit ohne den Einsatz von Lösungsmittel in eine Endkundenformulierung einfach eingearbeitet werden können, wodurch in dieser nicht zwangsläufig solch ein Lösungsmittel enthalten sein muss.

Noch ein Vorteil der vorliegenden Erfindung ist es, dass der Glanz der behandelten keratinischen Fasern gesteigert wird.

Ein weiterer Vorteil der vorliegenden Erfindung ist, dass die eingesetzten Verbindungen bereits bei geringen Einsatzmengen eine gute Wirkung entfalten.

Ein weiterer Vorteil ist, dass die eingesetzten Verbindungen in ökologischer Hinsicht wenig belastend sind.

Noch ein Vorteil ist es, dass die erfindungsgemäßen Formulierungen auf keratinischen Fasern einen verbesserten konditionierenden Effekt bei längeren Abspülzeiten zeigen als bisher bekannte quartäre Esterverbindungen.

Noch ein Vorteil der vorliegenden Erfindung ist es, dass Esterquats eine erhöhte Hydrolysestabilität in der Formulierung aufweisen.

Noch ein Vorteil der vorliegenden Erfindung ist es, dass sie nicht auskristallisieren. Noch ein Vorteil der vorliegenden Erfindung ist es, dass sie in geringeren Einsatzkonzentrationen wirksam sind.

Noch ein Vorteil der vorliegenden Erfindung ist es, dass die erfindungsgemäßen Formulierungen Haarfärbemittel vor dem Auswaschen schützen.

Noch ein Vorteil der vorliegenden Erfindung ist es, dass die erfindungsgemäßen Formulierungen keratinische Fasern gegenüber thermisch induzierten Schäden schützen.

Noch ein Vorteil der vorliegenden Erfindung ist es, dass die erfindungsgemäßen Formulierungen die Kammkräfte am nassen und am trockenen Haar reduzieren. Noch ein Vorteil der vorliegenden Erfindung ist es, dass die erfindungsgemäßen Formulierungen methanolfrei hergestellt werden können.

Noch ein Vorteil der vorliegenden Erfindung ist es, dass die erfindungsgemäßen Formulierungen besonders wirtschaftlich sind.

Unter dem Begriff "Esterquat" im Zusammenhang mit der vorliegenden Erfindung versteht man eine chemische Verbindung, die sowohl ein quartäres Stickstoffatom, als auch eine Esterbindung im kationischen Teil eines Ionenpaares enthält. Bevorzugt versteht man darunter eine Klasse von oberflächenaktiven quaternären Ammoniumverbindungen mit der allgemeinen Formel R₁₁R₁₂R₁₃R₁₄N + X-, dadurch gekennzeichnet, dass mindestens einer der Reste R₁₁ bis R₁₄, der dadurch gekennzeichnet ist, dass er mehr als 4 C-Atome aufweist an die geladene Gruppe über Esterbindungen C (O) O- oder OC(O)- gebunden ist, und unter X- jedes anionisches Gegenion verstanden wird.
- R₁₁: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Koh lenwasserstoffrest
- R₁₂: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Koh lenwasserstoffrest
- R₁₃: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Koh lenwasserstoffrest
- R₁₄: ein zweiwertiger, gesättigter oder ungesättigter, geradkettiger, verzweigter oder cyclischer, gegebenenfalls von Sauerstoff- oder Stickstoffatomen oder Carboxylgruppen unterbrochener und gegebenenfalls substituierter Kohlenwasserstoffrest

Unter dem Begriff "flüssige Esterquats" im Zusammenhang mit der vorliegenden Erfindung werden Esterquats, die bei 1 bar einen Schmelzpunkt von 25 °C oder kleiner verstanden. Handelt es sich bei den in der Formulierung enthaltenen Esterquats um Mischungen von Esterquats, so bezieht sich der Schmelzpunkt auf den Schmelzpunkt der Mischung aller in der Formulierung enthaltenen Esterquats. Analoges gilt für Imidazoliniumsalze, für Fettalkohole und Emulgatoren.

Unter dem Begriff "Polymerverdicker" im Zusammenhang mit der vorliegenden Erfindung werden Polymere verstanden, die die Viskosität wässriger Phasen signifikant erhöhen können.

Alle angegebenen Prozent (%) sind, wenn nicht anders angegeben, Massenprozent.

Die im Rahmen der Erfindung eingesetzten quaternierten Fettsäurealkoholaminester lassen sich nach einschlägigen Methoden der präparativen organischen Chemie herstellen. Üblicherweise beruht die Herstellung von Esterquats auf einem mehrstufigen Prozess, bei dem zunächst durch Umsetzung eines Alkanolamins mit Carbonsäuren oder entsprechenden Derivaten das veresterte Alkanolamin hergestellt wird, das dann im Anschluss mit einem geeigneten Reagens quaterniert wird.

In Zusammenhang für geeignete Herstellverfahren sei auf die EP0483195 verwiesen, nach der man Trialkanolamin in Gegenwart von unterphosphoriger Säure mit Fettsäuren partiell verestert, Luft durchleitet und anschließend mit Dimethylsulfat oder Ethylenoxid quaterniert. Die dort aufgeführten Verbindungen dienen als Weichmacher für Textilien. In der DE4308794 wird die Herstellung von Esterquats beschrieben, indem die Quaternisierung der Triethanolaminester in Gegenwart geeigneter Dispergiermittel durchgeführt wird. Übersichten zu dieser Thematik lassen sich zum Beispiel unter R. Puchta et al. in Tens. Surf. Det., 30, 186 (1993), M. Brock in Tens. Surf. Det., 30, 394 (1993), R. Lagerman et al. in J. Am. Chem. Soc., 71, 97 (1994) oder unter I. Shapiro in Cosm. Toil., 109, 77 (1994) nachlesen.

In der erfindungsgemäßen Formulierung enthaltene flüssige Esterquats umfassen mindestens eine Verbindung der allgemeinen Formel I) 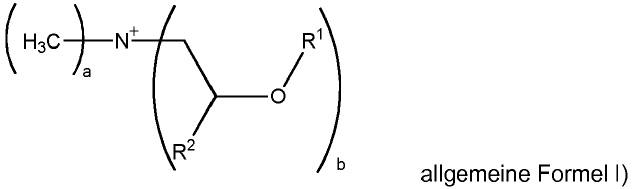 mit R¹ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3, bevorzugt a = 1,7 bis 2,3 und b = 1,7 bis 2,3
mit der Maßgabe, dass a + b =4.

Für den Fall, dass b>1 ist, können die Reste R¹ gleich oder ungleich sein.
R¹ als Acylrest einer mindestens einfach ungesättigten Fettsäure mit einer Kettenlänge von 18 bis 24 Kohlenstoffatomen kann eine oder mehr, beispielsweise zwei oder drei Doppelbindungen enthalten.
Erfindungsgemäß bevorzugte Formulierungen sind dadurch gekennzeichnet, dass R¹ als Acylrest einer mindestens einfach ungesättigten Fettsäure mit einer Kettenlänge von 18 bis 24 Kohlenstoffatomen ausgewählt ist aus den Acylresten der Säuren aus der Gruppe umfassend
Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, und Cervonsäure, wobei Ölsäure besonders bevorzugt ist.
Es können erfindungsgemäß auch Mischungen dieser Carbonsäuren eingesetzt werden.
Bevorzugte Formulierungen enthalten mindestens eine Verbindung der allgemeinen Formel I) mit a = 1,7 bis 2,3 und b = 1,7 bis 2,3, besonders bevorzugt a = b = 2.
Eine erfindungsgemäß besonders bevorzugte Formulierung ist dadurch gekennzeichnet, dass R¹ der Acylrest der Ölsäure und a = 1,7 bis 2,3 und b = 1,7 bis 2,3, besonders bevorzugt a = b = 2, ist.

In der erfindungsgemäßen Formulierung können neben einer Verbindung der allgemeinen Formel I) weitere Verbindungen enthalten sein, die bis auf den Rest R¹ der Verbindung der allgemeinen Formel I) entsprechen, das heißt der analoge Rest R^{1a} ist der Acylrest einer anderen Carbonsäure, insbesondere einer anderen Fettsäure. Somit kann neben der Verbindung der allgemeinen Formel I) auch mindestens eine Verbindung der allgemeinen Formel Ia) 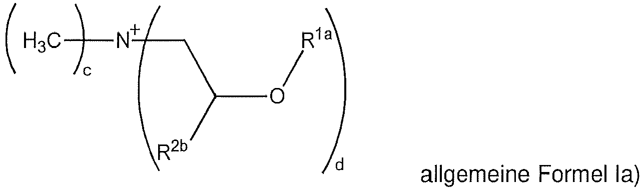 mit R^{1a} ein Acylrest einer anderen Carbonsäure als für R¹ definiert und
mit R^{2b} ein Alkylrest mit 1 bis 6 Kohlenstoffatomen, bevorzugt Methyl, Ethyl, Propyl, Isopropyl, besonders bevorzugt Methyl,
wobei c = 1 bis 3 und d = 1 bis 3,
mit der Maßgabe, dass c + d = 4,
enthalten sein.

Die Formulierungen enthalten als Komponente A) somit bevorzugt eine Mischung mindestens einer Verbindung der allgemeinen Formel I) und mindestens einer Verbindung der allgemeinen Formel Ia), wie sie sich beispielsweise bei dem Einsatz von technischen Fettsäureschnitten ergibt, welche längere oder kürzere Acylreste aufweisen, als für R¹ oben.

Die Verbindungen der allgemeinen Formel I) machen bevorzugt mindestens 30 Gew.-%, bevorzugt mindestens 50 Gew.-%, besonders bevorzugt mindestens 75 Gew.-%, bezogen auf alle Verbindungen der allgemeinen Formel I) und Ia) aus.

Ganz besonders bevorzugt sind Mischungen, die erhalten werden, wenn als Mischung Pflanzenmischöle mit einer C-Kettenverteilung eingesetzt werden, für die gilt: Kettenlänge von R¹ bzw. R^{1a} Anteil bezogen auf Gesamtmischung (' = Anzahl der Doppelbindung(en))

| | |
|---|---|
| <C 16 | 0 - 2 Gew.-% |
| C 16 | 4 - 7 Gew.-% |
| C 16' | 0 - 2 Gew.-% |
| C 18 | 0 - 4 Gew.-% |
| C 18' | 55 - 65 Gew.-% |
| C 18" | 15 - 25 Gew.-% |
| C 18''' | 6 - 112 Gew.-% |
| >C 18 | 0 - 4 Gew.-% |

Die Verbindungen der allgemeinen Formel I) mit R¹ = der Acylrest der Ölsäure machen bevorzugt mindestens 40 Gew.-%, besonders bevorzugt mindestens 55 Gew.-%, bezogen auf alle Verbindungen der allgemeinen Formel I) und Ia) aus.
Die Verbindungen der allgemeinen Formel I) mit R¹ = der Acylrest einer C16-Säure machen bevorzugt höchstens 20 Gew.-%, besonders bevorzugt höchstens 11 Gew.-%, bezogen auf alle Verbindungen der allgemeinen Formel I) und Ia) aus.

Bevorzugte Formulierungen dieser Ausführungsform enthalten Verbindungen der allgemeinen Formel I) bzw. Ia) mit a = b = c = d = 2.

Erfindungsgemäß bevorzugte Formulierungen, insbesondere solche zur Behandlung keratinischer Fasern, insbesondere von menschlichem Haar, enthalten 0,1 bis 7 Gew.-%, bevorzugt 0,2 bis 5 Gew.-% und insbesondere bevorzugt 0,3 bis 3 Gew.-%, Komponente A), wobei sich die Gew.-% auf die Gesamtformulierung beziehen.
Besonders gute Ergebnisse lassen sich bei den vordefinierten Konzentrationen von 0,2 bis 2 Gew.-% erzielen. Die Anwendung der erfindungsgemäßen Formulierungen an keratinischen Fasern, insbesondere an menschlichen Haaren ist jedoch nicht auf den Einsatz der Wirkstoffe in geringer Konzentration limitiert. Es können ebenso konzentrierte erfindungsgemäße Formulierungen zur Anwendung kommen, in denen die vordefinierten Konzentrationen 2 bis 20 Gew.-% oder 3 bis 14 Gew.-%, insbesondere 5 bis 12 Gew.-%, betragen.

Die Stoffgruppe der polymeren Verdicker umfasst die natürlichen Verdicker, wie Stärke, Gelatine, Alginate, abgewandelte Naturstoffe, wie Celluloseether oder Hydroxyethylcellulose, und die vollsynthetischen Polymere, wie z. B. Polyacrylate, oder Polyacrylamide sowie sogenannte HEUR = nonionic hydrophobically modified ethoxylated Urethane, auch bekannt als assoziative Polyurethanverdicker. Diese Polymere bestehen mindestens aus zwei kohlenwasserstoffbasierten lipophilen Polymerketten, die aus 6 bis 30 Kohlenstoffatomen aufgebaut sind und durch eine hydrophile Einheit verbrückt sind. Beispiele für solche Polyurethanverdicker sind PEG-150/stearyl alcohol/SDMI copolymer, PEG-150/ Decyl Alcohol/ SMDI Copolymer, Bis-Stearyl PEG/PPG-8/6 SMDI/PEG-400 Copolymer und Polyether-urea-polyurethane. Solche Stoffe sind kommerziell erhältlich unter den folgenden Produktnamen SERAD FX1010, SERAD FX1100 and SERAD FX1035 (kommerziell erhältlich von Hüls America), RHEOLATE 255, RHEOLATE 278 und RHEOLATE 244 (kommerziell erhältlich von Elementis), DW 1206F, DW 1206J, DW 1206B, DW 1206G, und ACRYSOL RM 2020
Weitere polymere Verdicker sind auf Basis von (Schicht-)Silikaten oder modifizierten Tonerden wie Bentonite oder Hectorite oder deren Derivaten bekannt.
Als Polymerverdicker in der erfindungsgemäßen Formulierung (Komponente B) lassen sich somit beispielsweise Cellulose, Xanthan, Carrageenan, Galactomannane, Guar, Tara, Cassia, Sesbania, Locust Bean Gum, Gellan Gum, Wellan Gum, Johannisbrotkernmehl, Guarkernmehl, Stärke, Pektin, Lanolin, Agar-Agar, Traganth, Polysaccharide (z.B. aus Algen, Bakterien oder Pilzen isoliert sowie solche gelistet in "Ullmanns Encyklopädie der technischen Chemie", Bd. 19, Verlag Chemie Weinheim, 1980, S. 233-263), Alginate, Carrageenan, Gellan, Pullulan, Scleroglucan, Schizophyllan, Curdlan, Diutan, Dextran, Welan, Chitin einsetzen, sowie Derivate davon, insbesondere Derivate in Form von Alkylierungen (z. B. Methylether, Ethylether, C12-18 Alkylether), Hydroxyalkylierungen (hydroxyethyl-, hydroxypropyl- oder Mischether) und Carboxymethylierung. Bevorzugte Derivate sind Stärkederivate, wie z.B. Hydroxypropyl Starch Phosphate, kommerziell erhältlich unter dem Namen Structure XL (Akzo Nobel). Darüber hinaus lassen sich in den erfindungsgemäßen Formulierungen vollsynthetische Polymere einsetzen wie z.B. polyacrylatbasierte Verdicker, Polyacrylamidverdicker, beispielsweise quervenetzte Copolymere (polyacrylamide/C13-14 isoparaffin/Laureth 7) (acrylamide/sodium acryloyldimethyltaurate copolymer/isohexadecane/polysorbate 80), oder lineare, amorphe sulfonierte Polyester, beispielsweise Polyester-4.
Darüber hinaus können auch kationische/kationisierte Polymere eingesetzt werden, wie z.B. Guar-quats, beispielsweise Guar Hydroxypropyltrimonium Chloride, Lanolin-Quats, quaternäre Derivate der Hydroxyethylcellulose, beispielsweise HEC Cocodimonium Chloride, hydrolysierte Proteine, beispielsweise Hydroxypropyltrimonium Hydrolyzed Wheat Protein oder Cassia Hydroxypropyltrimonium Chloride, oder Polyquaternium Polymere, beispielsweise Polyquaternium-2, Polyquaternium-4, Polyquaternium-6, Polyquaternium-7, Polyquaternium-10, Polyquaternium-20, Polyquaternium-22, Polyquaternium-24, Polyquaternium-37, Polyquaternium-39, Polyquaternium-47, Polyquaternium-53, Polyquaternium-59, Polyquaternium-67, Polyquaternium-70, Polyquaternium-72, Polyquaternium-74.
Generell können auch Mischungen aus unterschiedlichen Polymerverdickern eingesetzt werden.

Besonders bevorzugte erfindungsgemäße Formulierungen enthalten als Polymerverdicker mindestens einen ausgewählt aus der Gruppe der polyacrylatbasierten Verdicker, z.B die sogenannten Carbomere. Carbomere sind Polymere der Acrylsäure oder Copolymere aus C10-30-Alkylacrylaten und einem oder mehreren Monomeren der Acrylsäure, der Methacrylsäure oder deren Ester, die kreuzvernetzt sind, z.B. mit einem Allylether der Saccharose oder einem Allylether des Pentaerythrit.
Insbesondere sind die polyacrylatbasierten Verdicker ausgewählt aus Polymeren auf Basis von mindestens einem der folgenden Monomere: Acrylsäure und ihre Salze, Methacrylsäure und ihre Salze, Alkylester der Acrylsäure und Methacrylsäure (z.B. C2-C30), Hydroxyethyl Acrylate, Acrylamid, 2-Acrylamido-2-Methylpropansulfonsäure und ihren Salzen (AMPS). Insbesondere handelt es sich bei den polyacrylatbasierten Verdickern dabei um Produkte mit den INCI Bezeichnungen ausgewählt aus Carbomer, Sodium Carbomer, Acrylates/C10-C30 Alkyl Acrylate Crosspolymer, Sodium Acrylates/Beheneth-25 Methacrylate, acrylates/C12-24 pareth-25 acrylate copolymer, acrylates/steareth-20 methacrylate copolymer, acrylates/beheneth-25 methacrylate copolymer, acrylates/steareth-20 itaconate copolymer, acrylates/aminoacrylates/C10-30 alkyl PEG-20 itaconate copolymer, Acrylates Copolymer, Acrylates Crosspolymer-4, Ammonium Acrylates Copolymer, Polyacrylate-1 Crosspolymer, Acrylamide / Sodium Acryloyldimethyl Taurate Copolymer, Sodium Acrylate / Sodium Acryloyldimethyl Taurate Copolymer, Acrylic Acid / Vinyl Pyrrolidone, Acrylates/Vinyl Neodecanoate Crosspolymer, Polyacrylamide/C13-14 Isoparaffin/Laureth-7, Acrylamide/Sodium acryloyldimethyltaurate polymer/Isohexadecane/Polysorbate 80 oder Sodium Acrylate / Acryloyldimethyltaurate / Dimethylacrylamide Crosspolymer. Beispiele der Polyacrylatverdicker sind Polyacrylate aus der Gruppe der sogenannten Carbopole, beispielsweise Carbopole der Typen 980, 981, 1382, 2020, 2984, 3128, 5984 und Pemulen TR1 , TR2, jeweils einzeln oder in Kombination.

Bevorzugt enthält die erfindungsgemäße Formulierung die Polymerverdicker in einer Menge von 0,005 Gew.-% bis 2 Gew.-%, bevorzugt 0,01 Gew.-% bis 0,5 Gew.-%, besonders bevorzugt 0,05 Gew.-% bis 0,1 Gew.-%, bezogen auf die Gesamtformulierung.

Erfindungsgemäße Formulierungen sind Emulsionen, bevorzugt Öl in Wasser Emulsionen, besonders bevorzugt Emulsionen, bei denen die Ölphase bei 25 °C fest ist, somit liegt bei 25 °C eine Öl-in-Wasser Suspension vor.

Es hat sich als vorteilhaft erwiesen, wenn die erfindungsgemäßen Formulierungen zusätzlich mindestens einen Fettalkohol (Komponente C)) enthalten.
Als Fettalkohol wird in diesem Zusammenhang bevorzugt ein unverzweigter oder verzweigter Monoalkohol mit einer Alkylgruppe von 8 bis 30 C-Atomen verstanden, der auch ungesättigt sein kann. Bevorzugte Fettalkohole sind Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol,lsostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol sowie Mischungen davon, insbesondere technische Mischungen, vorzugsweise technische Kokos- oder Talgfettalkohole mit 12 bis 18, vorzugsweise mit 16 bis 18 Kohlenstoffatomen, sowie die einfach ungesättigten Fettalkohole, wie Oleylalkohol, Elaidylalkohol, Delta-9-cis-Hexadecenol, Delta-9-Octadecenol, trans-Delta-9-Octadecenol, cis-Delta-11-Octadecenol, trans-10,cis-12-Hexadecadien-1-ol, Octacosa-10,19-dien-1-ol und mehrfach ungesättigte Fettalkohole wie z.B. Linoleylalkohol (9Z, 12Z-octadecadien-1-ol), Elaidolinoleylalkohol (9E, 12E-octadecadien-1-ol), Linolenylalkohol (9Z, 12Z, 15Z-octadecatrien-1-ol), Elaidolinolenylalkohol (9E, 12E, 15-E-octadecatrien-1-ol), wobei Mischungen von Kokos- oder Talgfettalkohole mit 16 bis 18 Kohlenstoffatomen besonders bevorzugt sind.
Bevorzugt enthält Komponente C) mindestens einen Fettalkohol, der einen Schmelzpunkt von größer 25 °C, besonders bevorzugt größer 50 °C, bei 1 bar Druck aufweist.
Der Fettalkohol ist bevorzugt in einer Menge von 0,5 bis 20 Gew.-%, bevorzugt 1 bis 10 Gew.-%, insbesondere 2 bis 7 Gew. %, enthalten, wobei sich die Gewichtsprozente auf die Gesamtformulierung beziehen.

Bevorzugte erfindungsgemäße Formulierungen sind dadurch gekennzeichnet, dass Komponente A) in einer Menge von 0,1 bis 7 Gew.-%, bevorzugt 0,2 bis 5 Gew.-% und insbesondere bevorzugt 0,3 bis 3 Gew.-%,
Komponente B) in einer Menge von 0,005 Gew.-% bis 2 Gew.-%, bevorzugt 0,01 Gew.-% bis 0,5 Gew.-%, besonders bevorzugt 0,05 Gew.-% bis 0,1 Gew.-%, bezogen auf die Gesamtformulierung, und
Komponente C) in einer Menge von 0,5 bis 20 Gew.-%, bevorzugt 1 bis 10 Gew.-%, insbesondere 2 bis 7 Gew. %, bezogen auf die Gesamtformulierung, enthalten ist.

In einer besonders bevorzugten alternativen Ausführungsform ist Komponente B) ausgewählt aus den assoziativen Polyurethanverdickern und die Formulierung enthält keine Komponente C), ist somit frei von Fettalkohol. In diesem Zusammenhang sind die assoziativen Polyurethanverdicker bevorzugt, die mindestens aus zwei kohlenwasserstoffbasierten lipophilen Polymerketten bestehen, die aus 6 bis 30 Kohlenstoffatomen aufgebaut sind und durch eine hydrophile Einheit verbrückt sind. Beispiele für solche Polyurethanverdicker sind PEG-150/stearyl alcohol/SDMI copolymer, PEG-150/ Decyl Alcohol/ SMDI Copolymer, Bis-Stearyl PEG/PPG-8/6 SMDI/PEG-400 Copolymer und Polyether-urea-polyurethane. Solche Stoffe sind kommerziell erhältlich unter den folgenden Produktnamen SERAD FX1010, SERAD FX1100 und SERAD FX1035, RHEOLATE 255, RHEOLATE 278 und RHEOLATE 244, DW 1206F, DW 1206J, DW 1206B, DW 1206G, und ACRYSOL RM 2020.
Bevorzugt enthalten die erfindungsgemäßen Formulierungen dieser alternativen Ausführungsform
Komponente A) in einer Menge von 0,1 bis 7 Gew.-%, bevorzugt 0,2 bis 5 Gew.-% und insbesondere bevorzugt 0,3 bis 3 Gew.-%,
Komponente B) in einer Menge von 0,005 Gew.-% bis 30 Gew.-%, bevorzugt 0,5 Gew.-% bis 15 Gew.-%, besonders bevorzugt 1 Gew.-% bis 5 Gew.-%, bezogen auf die Gesamtformulierung.

Es hat sich als vorteilhaft erwiesen, wenn die erfindungsgemäßen Formulierungen zusätzlich mindestens einen Emulgator (Komponente D)), enthalten, bevorzugt in einer Menge von 0,1 bis 10 Gew.-%, bevorzugt 0,25 bis 5 Gew.-%, insbesondere 0,5 bis 2,5 Gew.-%, wobei sich die Gewichtsprozente auf die Gesamtformulierung beziehen.
In diesem Zusammenhang bevorzugte Emulgatoren sind ausgewählt aus der Gruppe der Fettalkoholalkoxylate, insbesondere der Fettalkoholethoxylate. Besonders bevorzugt enthaltene Fettalkoholethoxylate sind ausgewählt aus der Gruppe umfassend Polyoxyethylenether des Laurylalkohols, CAS-Nummer 9002-92-0, Macrogollaurylether, z. B. Polyoxyethylen (4) laurylether (Laureth-4, INCI), Polyoxyethylen (9) laurylether Laureth-9 (INCI),
Polyoxyethylen (23) laurylether Laureth-23 (INCI)
Polyoxyethylenether des Cetylalkohols, CAS-Nummer 9004-95-9, z.B.
Polyoxyethylen (2) cetylether Ceteth-2 (INCI),
Polyoxyethylen (10) cetylether Ceteth-10 (INCI)
Polyoxyethylen (20) cetylether Ceteth-20 (INCI)
Polyoxyethylenether des Cetylstearylalkohols, CAS-Nummer 68439-49-6, z.B.
Polyoxyethylen (6) cetylstearylether Ceteareth-6 (INCI)
Polyoxyethylen (20) cetylstearylether Ceteareth-20 (INCI)
Polyoxyethylen (25) cetylstearylether Ceteareth-25 (INCI)
Polyoxyethylenether des Stearylalkohols, CAS-Nummer 9005-00-9, z.B.
Polyoxyethylen (2) stearylether Steareth-2 (INCI)
Polyoxyethylen (10) stearylether Steareth-10 (INCI)
Polyoxyethylen (20) stearylether Steareth-20 (INCI)
Polyoxyethylenether des Oleylalkohols, CAS-Nummer 9004-98-2, z.B.
Polyoxyethylen (2) oleylether Oleth-2 (INCI)
Polyoxyethylen (10) oleylether Oleth-10 (INCI)
Polyoxyethylen (20) oleylether Oleth-20 (INCI)
oder
Polyoxyethylen (10) tridecylether (CAS-Nummer 24938-91-8) und Trideceth-10 (INCI). Alternativ bevorzugte Emulgatoren sind ausgewählt aus der Gruppe der Polyolester, insbesondere der Glycerinester und Polyglycerinester, insbesondere der Polyglycerinester. Bevorzugt enthaltene (Poly)glycerinester sind dadurch gekennzeichnet, dass sie Partialester sind. Besonders bevorzugte Polyglycerinpartialester sind ausgewählt aus der Gruppe umfassend Polyglycerinpartialester wie in EP-B-0 835 862 beschrieben, die erhältlich sind durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4, Ester der Zitronensäure wie etwa der O/W-Emulgator Glycerylstearatcitrat, (2-Hydroxy-1,2,3-propantricarbonsäure-1,2,3-propantriolmonooctadecanoat, INCI Glyceryl Stearate Citrate, CAS 39175-72-9), der Citronensäureester des Glycerylstearats, u.a. unter der Bezeichnung AXOL C 62 im Handel erhältlich, Glycerylstearatcitrat wie in WO2006034992 und WO2008092676 beschrieben und Glyceryloleatcitrat wie in WO2004112731 beschrieben, ebenso einfache Polyglycerinester, wie beispielsweise Polygylcerin-3 Distearate, Polyglyceryl-10 Stearate, Polyglyceryl-6 Distearate, Mischester aus Polyglycerin und Methylglucose und Stearinsäure, wie beispielsweise Polyglyceryl-3 Methylglucose Distearate und (Poly-)glycerinpartialester mit einer oder mehrerer Carbonsäuren mit 10 bis 24 Kohlenstoffatomen und Resten einer polyfunktionalen Carbonsäure.
Prinzipiell können auch Sorbitan- oder Saccharoseester als Polyolester eingesetzt werden. Eine gängige Kombination ist beispielsweise Sorbitan Stearate & Sucrose Cocoate.
In einer weiteren Alternative bevorzugt enthaltene Emulgatoren sind ausgewählt aus der Gruppe der modifizierten Siloxane, beispielsweise solche, die neben aliphatischen Gruppen auf Basis von alpha-Olefinen auch Polyether tragen. Siloxanbasierte Emulgatoren für Öl-in-Wasser-Emulsionen müssen einen hydrophilen Charakter aufweisen, weshalb es sich in der Regel um reine Polyethersiloxane handelt. Besonders geeignete Beispiele sind relativ hydrophobe Polyethersiloxane wie in EP 1125574 beschrieben, hochmolekulare Polyethersiloxane wie in EP2168564 beschrieben und organomodifizierte Siloxanblockcopolymere wie in WO2009138306 beschrieben. Bevorzugt enthaltene modifizierte Siloxane sind dadurch gekennzeichnet, dass sie einen HLB-Wert >8 aufweisen. Besonders bevorzugte modifizierte Siloxane sind ausgewählt aus der Gruppe umfassend Bis-PEG/PPG-16/16 Dimethicone, PEG/PPG-16/16 Dimethicone, Bis-PEG/PPG-20/5 PEG/PPG-20/5 Dimethicone und Methoxy PEG/PPG-25/4 Dimethicone.
Vorgenannte Emulgatoren ergeben im Zusammenhang mit der vorliegenden Erfindung besonders lagerstabile Formulierungen.
Es ist insbesondere vorteilhaft und bevorzugt, wenn als Komponente D) Emulgatoren enthalten sind, die bei 1 bar einen Schmelzpunkt von 60 °C oder kleiner, bevorzugt 40 °C und kleiner, besonders bevorzugt 25 °C oder kleiner und ganz besonders bevorzugt 10 °C oder kleiner aufweisen.
Fettalkohole sind keine Emulgatoren im Sinne der vorliegenden Erfindung.

Bevorzugte erfindungsgemäße Formulierungen sind dadurch gekennzeichnet, dass Komponente A) in einer Menge von 0,1 bis 7 Gew.-%, bevorzugt 0,2 bis 5 Gew.-% und insbesondere bevorzugt 0,3 bis 3 Gew.-%,
Komponente B) in einer Menge von 0,005 Gew.-% bis 2 Gew.-%, bevorzugt 0,01 Gew.-% bis 0,5 Gew.-%, besonders bevorzugt 0,05 Gew.-% bis 0,1 Gew.-%, bezogen auf die Gesamtformulierung,
Komponente C) in einer Menge von 0,5 bis 20 Gew.-%, bevorzugt 1 bis 10 Gew.-%, insbesondere 2 bis 7 Gew. %, und
Komponente D) in einer Menge von 0,1 bis 10 Gew.-%, bevorzugt 0,25 bis 5 Gew.-%, insbesondere 0,5 bis 2,5 Gew.-%,
jeweils bezogen auf die Gesamtformulierung, enthalten ist.

Eine bevorzugte alternative Ausführungsform ist kalt herstellbar, d.h. sie enthält nur Komponenten die ohne Aufschmelzen verarbeitet werden können.
Diese erfindungsgemäßen Formulierungen sind dadurch gekennzeichnet, dass alle enthaltenen Esterquats, Imidazoliniumsalze, Fettalkohole und Emulgatoren bei 1 bar einen Schmelzpunkt von 60 °C oder kleiner, bevorzugt 40 °C und kleiner, besonders bevorzugt 25 °C oder kleiner und ganz besonders bevorzugt 10 °C oder kleiner aufweisen, wobei für den Fall, dass mehr als ein Esterquat, Imidazoliniumsalz, Fettalkohol oder Emulgator enthalten ist, sich der Schmelzpunkt auf die Mischung von jeweils allen Esterquats, Imidazoliniumsalzen, Fettalkoholen oder Emulgatoren bezieht.

Die erfindungsgemäßen Formulierungen können z.B. mindestens eine weitere, zusätzliche Komponente enthalten, ausgewählt aus der Gruppe der
Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der deutschen Anmeldung DE 102008001788.4 entnommen werden.

Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosemtika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Es ist erfindungsgemäß bevorzugt, wenn die Formulierung einen pH-Wert von 3,0 bis 5,5 aufweist, bevorzugt 3,5 bis 5,0.

Die Ladung der in der erfindungsgemäßen Formulierung enthaltenen Esterquats muss durch entsprechende Anionen kompensiert werden; dies findet durch in der erfindungsgemäßen Formulierung enthaltene Gegenanionen statt.

Als solche Gegenanionen sind beispielsweise die Halogenide, Pseudohalogenide, Anionen von Mineralsäuren, Sulfate, Sulfite, Hydrogensulfite, Sulfonat, Alkyl- und Arylsulfonate, Phosphat, Hydrogenphosphate, Phosphite, Hydrogenphosphite, Phosphonite, Carboxylate, Borate, Carbonate, Sulfide, Hydrogensulfide, Lactat, Glycolat, Formiat, Acetat und Propionat geeignet.

Diese Anionen sind bevorzugt ausgewählt aus solchen, die für kosmetische Anwendung geeignet und somit beispielsweise nicht toxisch sind. Besonders bevorzugt ist mindestens ein Gegenanion zu dem Esterquat ausgewählt aus der Gruppe umfassend Chlorid, Bromid, lodid, Alkylsulfat, z.B. Methylsulfat, Ethylsulfat, Alkylsulfonat, z.B. Methylsulfonat, Triflat, Tosylat, Phosphat, Sulfat, Hydrogensulfat, Lactat, Glycolat, Acetat und Citrat, bevorzugt Chlorid und Methylsulfat, enthalten.

Die erfindungsgemäßen Formulierungen lassen sich erfindungsgemäß für die Behandlung keratinischer Fasern, insbesondere für die Haarbehandlung, verwenden. Die erfindungsgemäße Verwendung führt zur Verbesserung der Konditionierung, des Glanzes, der Flexibilität, der Spannkraft, und/oder der Kämmbarkeit sowie einer Reduzierung der Bruchwahrscheinlichkeit der behandelten Faser und außerdem reduziert sie die antistatischen Kräfte zwischen den Fasern.

Die erfindungsgemäße Verwendung führt zum Schutz der Fasern vor Hitze.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Synthesebeispiel 1: Herstellung von "1-Propanaminium, 2-hydroxy-N-(2-hydroxypropyl)-N,N-dimethyl-, diester mit Pflanzenmischölfettsäure, Methylsulfat" (erfindungsgemäß)

1120 g (4 mol) Pflanzenmischölfettsäure werden mit 302 g (2,05 mol) Methyldiisopropanolamin gemischt unter Rühren auf 180 °C aufgeheizt. Reaktionswasser wird kontinuierlich abdestilliert. Nachdem die Hauptmenge Reaktionswassser bei Normaldruck destilliert ist, wird Vakuum angelegt und die Säurezahl der Reaktionsmischung auf < 7 mg KOH/g abreagiert. Das entstandene Esteramin wird auf 60 °C abgekühlt und portionsweise mit 240 g (1,90 mol) Dimethylsulfat versetzt, so dass die Reaktionstemperatur 100 °C nicht übersteigt. Nach dem Abkühlen auf Raumtemperatur wird die Gesamt Amin Zahl (GAZ) und der Aktivgehalt des Fertigprodukts analysiert. GAZ = 5,0 mg KOH/g; Aktivgehalt 1,27meq/g (Kationischer Aktivgehalt nach Epton).

### Synthesebeispiel 3: Herstellung von "1-Propanaminium, 2-hydroxy-N-(2-hydroxypropyl)-N,N-dimethyl-, diester mit Tallölfettsäure, Methylsulfat" (erfindungsgemäß)

814 g (2,84 mol) Tallölfettsäure werden mit 214 g (1,46 mol) Methyldiisopropanolamin gemischt und wie unter Beispiel 1 beschrieben verestert. Das Esteramin hatte eine Säurezahl von 3,2 mg KOH/g. Diese Mischung wurde wie in Beispiel 1 beschrieben mit 168 g (1,33 mol) Dimethylsufat alkyliert. Die GAZ des Fertigprodukts wurde mit 7,3 mg KOH/g bestimmt, der Aktivgehalt war 1,24 meq/g.

### Synthesebeispiel 4: Herstellung von "1-Propanaminium, 2-hydroxy-N-(2-hydroxypropyl)-N,N-dimethyl-, diester mit Erucasäure, Methylsulfat" (erfindungsgemäß)

392 g (1,15mol) Erucasäure werden mit 99,3 g (0,68mol) Methyldiisopropanolamin gemischt und wie unter Beispiel 1 beschrieben verestert. Das Esteramin hatte eine Säurezahl von 2,2 mg KOH/g. Diese Mischung wurde wie in Beispiel 1 beschrieben mit 72,2 g (0,57mol) Dimethylsufat alkyliert. Die GAZ des Fertigprodukts wurde mit 4,6 mg KOH/g bestimmt, der Aktivgehalt war 1,45meq/g.

### Synthesebeispiel 5: Herstellung von "1-Propanaminium, 2-hydroxy-N-(2-hydroxypropyl)-N,N-dimethyl-, diester mit Isostearinsäure, Methylsulfat" (erfindungsgemäß)

1017 g (3,5 mol) Isostearinsäure (technische Qualität) werden mit 262 g (1,79 mol) Methyldiisopropanolamin versetzt und wie unter Beispiel 1 beschrieben verestert. Das Esteramin hatte eine Säurezahl von 3,4 mg KOH/g. Diese Mischung wurde wie unter Beispiel 1 beschrieben mit 209 g (1,66 mol) Dimethylsulfat alkyliert.

Die GAZ des Fertigprodukts wurde mit 5,1 mg KOH/g bestimmt, der Aktivgehalt war 1,26 meq/g.

### Anwendungstechnisches Beispiel 1: Stabilitäten der Formulierungsbeispiele (FB):

**Tab. 1a: Überblick der Formulierungsbeispiele für Stabilitätstests.**

| | FB 1 | FB 2 | FB 3 | FB 4 | FB 5 | FB 6 | FB 7 | FB 8 | FB 9 |
|---|---|---|---|---|---|---|---|---|---|
| TEGO® Alkano 1618, Evonik Industries AG (INCI: Cetearyl Alcohol) | 5,0% | 5,0% | 5,0% | 5,0% | 5,0% | 5,0% | 5,0% | 5,0% | 5,0% |
| TEGINACID® C, Evonik | 0,5% | 0,5% | 0,5% | 0,5% | 0,5% | 0,5% | 0,5% | 0,5% | 0,5% |
| Industries AG (INCI: Ceteareth-25) | | | | | | | | | |
| Synthesebeispiel1 | 1,0% | 1,0% | 1,0% | 1,0% | 1,0% | 1,0% | 1,0% | 1,0% | 1,0% |
| Oxynex® LM | 0,1% | 0,1% | 0,1% | 0,1% | 0,1% | 0,1% | 0,1% | 0,1% | 0,1% |
| Water | Ad 100 % | Ad 100% | Ad 100% | Ad 100% | Ad 100% | Ad 100% | Ad 100% | Ad 100% | Ad 100% |
| Preservative, Perfume | q.s. | q.s. | q.s. | q.s. | q.s. | q.s. | q.s. | q.s. | q.s. |
| TEGO® Carbomer 841 SER (Acrylates/ C10-30 Alkyl Acrylate Cross polymer ) | | 0,05 % | | | | | | | |
| TEGO® Carbomer 750 HD (Acrylates/ C10-30 Alkyl Acrylate Crosspolymer ) | | | 0,05 % | | | | | | |
| TEGO® Carbomer 341 ER (Acrylates/ C10-30 Alkyl Acrylate Crosspolymer ) | | | | 0,05 % | | | | | |
| TEGO® Carbomer 141 (Carbomer) | | | | | 0,05 % | | | | |
| Carrageen | | | | | | 0,05 % | | 0,025 % | |
| Xanthan Gum | | | | | | | 0,05 % | 0,025 % | |
| Hyd roxyethylcellulose | | | | | | | | | 0,05 % |

**Tab. 1b: Stabilität der Formulierungsbeispiele.**

| Formulierungsbeispiel | Viskosität Brookfield Tag 1 | Stabilität nach 3 Monaten RT | Stabilität nach 6 Monaten RT | Stabilität nach 9 Monaten RT | Stabilität nach 3 Monaten 45 °C |
|---|---|---|---|---|---|
| 1 (ohne Polymerverdicker) | + | - | -- | Phasentrennung | Phasentrennung |
| 2 (mit Polymerverdicker) | + | + | + | + | + |
| 3 (mit Polymerverdicker) | + | + | + | + | + |
| 4 (mit Polymerverdicker) | + | + | + | + | + |
| 5 (mit Polymerverdicker) | + | + | + | + | + |
| 6 (mit Polymerverdicker) | + | + | + | + | + |
| 7 (mit Polymerverdicker) | + | + | + | + | + |
| 8 (mit Polymerverdicker) | + | + | + | + | + |
| 9 (mit Polymerverdicker) | + | + | + | + | + |

| | | | | | |
|---|---|---|---|---|---|
| - -- = < 2000 mPas - - = > 2000 mPas < 7000 mPas - o = > 7000 < 10000 mPas - + = > 10000 mPas | | | | | |

Die Proben der Formulierungen wurden bei Raumtemperatur bzw. 45 °C gelagert. Die Viskositäten wurden mit einem Brookfield-Viskosimeter Typ LV-DV-I+ oder LV-DV-II+ bestimmt.)
Die Messung erfolgt bei 25 °C (+/- 0,5 °C). Zur Messung werden 100 ml Pulverflaschen gefüllt; temperiert und luftblasenfrei gemessen. Vor der Messung wird das Viskosimeter ohne Spindel durch Betätigen der Taste "Auto Range" kalibriert. Der Fortgang der Kalibration wird am Display angezeigt. Nach Abschluss der Kalibration wird eine Spindel entsprechend der zu erwartenden Viskosität am Viskosimeter montiert. Die Nummer der Spindel wird am Gerät eingegeben. Zur Bestimmung der Viskosität wird das Viskosimeter so zur Probe positioniert, dass die Spindel bis zur Markierung im Produkt eintaucht. Die Messung wird mit Hilfe der Starttaste ausgelöst. Die Messung sollte im günstigen Messbereich von 50% (+/- 20%) des maximal messbaren Drehmoments erfolgen. Das lässt sich durch Ändern der Umdrehungszahl / Wahl der Spindel einstellen.
Das Ergebnis der Messung wird am Display des Viskosimeters in mPas angegeben.

### Anwendungstechnisches Beispiel 2: Anwendungstechnik von Haarbehandlungsmitteln zur Beurteilung des Einflusses des Verdickers auf die sensorischen Eigenschaften der Formulierungen

Für die anwendungstechnische Beurteilung wurden Haartressen verwendet, die durch eine Bleichbehandlung standardisiert vorgeschädigt wurden. Dazu werden friseurübliche Produkte eingesetzt. Die Schädigung der Haartressen wird im Detail in der DE10327871 beschrieben.
Für die anwendungstechnische Beurteilung wurde die Beispielformulierung 1, welche keine polymeren Verdicker enthält mit den Beispielformulierungen 2-5 verglichen, welche zusätzlich einen polymeren Verdicker enthalten.

Die Zusammensetzung der Testformulierungen ist zur Vermeidung der Beeinflussung der Testergebnisse durch (normalerweise vorhandene) Formulierungsbestandteile bewusst einfach gewählt. Erfindungsgemäße Formulierungen können neben den genannten Inhaltsstoffen und/oder anstatt der genannten Inhaltsstoffe noch weitere Inhaltsstoffe enthalten. Insbesondere die Kombination mit weiteren Inhaltsstoffen kann bei den beschriebenen Effekten zu einer synergistischen Verbesserung führen.
Die Vorbehandlung der Haare erfolgt durch eine Shampooformulierung (Tab. 1), welche keine Konditioniermittel enthält.

**Tab. 2: Shampooformulierung für die Vorbehandlung der Haartressen.**

| | |
|---|---|
| Texapon NSO®, 28%-ig, Cognis (INCI: SodiumLaureth Sulfate) | 42,9% |
| NaCl | 3% |
| Wasser, demineralisiert | ad 100,0 |

Standardisierte Behandlung von vorgeschädigten Haarsträhnen mit konditionierenden Proben:
Die wie oben beschrieben vorgeschädigten Haarsträhnen werden mit der Shampooformulierung aus Tab. 2 gewaschen.
Hierbei werden die Haarsträhnen unter fließendem, warmem Wasser benetzt. Das überschüssige Wasser wird leicht von Hand ausgedrückt, dann wird das Shampoo aufgebracht und 1 min lang sanft ins Haar eingearbeitet (0.5 ml/2 g Haarsträhne). Die Haartresse wird für 30 s unter fließendwarmem Wasser ausgespült. Diese Prozedur wird ein weiteres Mal wiederholt, nur dass abschließend 1 min lang ausgespült wird. Anschließend werden direkt nach dem Waschen die Haarsträhnen mit den Formulierungsbeispielen 1-5 konditioniert.
Hierbei wird die Spülung aufgebracht und sanft ins Haar eingearbeitet (0.5 ml/2 g Haarsträhne). Nach einer Verweilzeit von 1 min wird das Haar für a) 1 min oder für b) 3 min gespült.
Vor der sensorischen Beurteilung wird das Haar an der Luft bei 50% Luftfeuchtigkeit und 25°C für mindestens 12 h getrocknet.

### Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.

### Die Testkriterien sind:

Nasskämmbarkeit, Nassgriff, Trockenkämmbarkeit, Trockengriff.

### a) 1 min Ausspülzeit

**Tab. 3a: Ergebnisse der Konditionierung von Haar bei 1 min Ausspülzeit**

| | Nasskämmbarkeit | Nassgriff | Trockenkämmbarkeit | Trockengriff |
|---|---|---|---|---|
| Formulierungsbeispiel 1 | 5 | 5 | 5 | 5 |
| Formulierungsbeispiel 2 | 5 | 5 | 5 | 5 |
| Formulierungsbeispiel 3 | 5 | 5 | 5 | 5 |
| Formulierungsbeispiel 4 | 5 | 5 | 5 | 5 |
| Formulierungsbeispiel 5 | 5 | 5 | 5 | 5 |

### b) 3 min Ausspülzeit

**Tab. 3b: Ergebnisse der Konditionierung von Haar bei 3 min Ausspülzeit**

| | Nasskämmbarkeit | Nassgriff | Trockenkämmbarkeit | Trockengriff |
|---|---|---|---|---|
| Formulierungsbeispiel 1 | 5 | 5 | 5 | 5 |
| Formulierungsbeispiel 2 | 5 | 5 | 5 | 5 |
| Formulierungsbeispiel 3 | 5 | 5 | 5 | 5 |
| Formulierungsbeispiel 4 | 5 | 5 | 5 | 5 |
| Formulierungsbeispiel 5 | 5 | 5 | 5 | 5 |

Die Ergebnisse in Tabelle 3a und 3b zeigen, dass die verbesserte Stabilität der Formulierungen die ausgezeichnete Haarkonditionierung nicht negativ beeinflusst.

### Formulierungsbeispiel 1 (nicht erfindungsgemäß)

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| Water | 93,4% |
| Preservative, Perfume | q.s. |

### Formulierungsbeispiel 2

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| TEGO® Carbomer 841 SER (Acrylates/ C10-30 Alkyl Acrylate Crosspolymer) | 0,05% |
| Water | 93,35% |
| Preservative, Perfume | q.s. |

### Formulierungsbeispiel 3

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| TEGO® Carbomer 750 HD (Acrylates/ C10-30 Alkyl Acrylate Crosspolymer) | 0,05% |
| Water | 93,35% |
| Preservative, Perfume | q.s. |

### Formulierungsbeispiel 4

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| TEGO® Carbomer 341 ER (Acrylates/ C10-30 Alkyl Acrylate Crosspolymer) | 0,05% |
| Water | 93,35% |
| Preservative, Perfume | q.s. |

### Formulierungsbeispiel 5

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| TEGO® Carbomer 141 (Carbomer) | 0,05% |
| Water | 93,35% |
| Preservative, Perfume | q.s. |

### Formulierungsbeispiel 6 (nicht erfindungsgemäß)

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| Carrageen | 0,05% |
| Water | 93,35% |
| Preservative, Perfume | q.s. |

### Formulierungsbeispiel 7

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| Xantham Gum | 0,05% |
| Water | 93,35% |
| Preservative, Perfume | q.s. |

### Formulierungsbeispiel 8

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| Xantham Gum | 0,025% |
| Carrageen | 0,025% |
| Water | 93,35% |
| Preservative, Perfume | q.s. |

### Formulierungsbeispiel 9

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 1,0% |
| Synthesebeispiel1 | 1,0% |
| Oxynex® LM | 0,1% |
| Hydroxyethylcellulose | 0,1% |
| Propyl Gallate | 0,1% |
| Water | 92,7% |
| Preservative, Perfume | q.s. |

### Formulierungsbeispiel 10

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 1,0% |
| Synthesebeispiel1 | 1,0% |
| Oxynex® LM | 0,1% |
| Hydroxyethylcellulose | 0,1% |
| Propyl Gallate | 0,1% |
| TEGIN® M (Glyceryl Stearate) | 2,0 % |
| Water | 90,7% |
| Preservative, Perfume | q.s. |

### Formulierungsbeispiel 11

| | |
|---|---|
| TEGO®Alkanol 1618, Evonik Industries AG (INCI: Cetearyl Alcohol) | 5,0% |
| TEGINACID® C, Evonik Industries AG (INCI: Ceteareth-25) | 0,5% |
| Synthesebeispiel1 | 1,0% |
| Oxynex® LM | 0,1% |
| TEGO® Carbomer 341 ER (Acrylates/ C10-30 Alkyl Acrylate Crosspolymer) | 0,1% |
| Water | 93,30% |
| Preservative, Perfume | q.s. |

### Formulierungsbeispiel 12 (nicht erfindungsgemäß)

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| Oxynex® LM | 0,1% |
| Water | 91,4% |
| Preservative, Perfume | q.s. |

### Formulierungsbeispiel 13

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| TEGO® Carbomer 841 SER (Acrylates/ C10-30 Alkyl Acrylate Crosspolymer) | 0,05% |
| Water | 91,45% |
| Preservative, Perfume | q.s. |

### Formulierungsbeispiel 14

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| TEGO® Carbomer 750 HD (Acrylates/ C10-30 Alkyl Acrylate Crosspolymer) | 0,05% |
| Water | 91,45% |
| Preservative, Perfume | q.s. |

### Formulierungsbeispiel 15

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| TEGO® Carbomer 341 ER (Acrylates/ C10-30 Alkyl Acrylate Crosspolymer) | 0,05% |
| Water | 91,45% |
| Preservative, Perfume | q.s. |

### Formulierungsbeispiel 16

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| TEGO® Carbomer 141 (Carbomer) | 0,05% |
| Water | 91,45% |
| Preservative, Perfume | q.s. |

### Formulierungsbeispiel 17

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| Carrageen | 0,05% |
| Water | 91,45% |
| Preservative, Perfume | q.s. |

### Formulierungsbeispiel 18

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| Xantham Gum | 0,1% |
| Water | 91,45% |
| Preservative, Perfume | q.s. |

### Formulierungsbeispiel 19

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| Xantham Gum | 0,05% |
| Carrageen | 0,05% |
| Water | 91,45% |
| Preservative, Perfume | q.s. |

### Formulierungsbeispiel 20

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel1 | 1,0% |
| Hydroxyethylcellulose | 0,1% |
| Propyl Gallate | 0,1% |
| Water | 90,8% |
| Preservative, Perfume | q.s. |

### Formulierungsbeispiel 21

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel1 | 1,0% |
| Hydroxyethylcellulose | 0,1% |
| Propyl Gallate | 0,1% |
| TEGIN® M (Glyceryl Stearate) | 2,0 % |
| Water | 88,8% |
| Preservative, Perfume | q.s. |

### Formulierungsbeispiel 22

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel1 | 1,0% |
| TEGO® Carbomer 341 ER (Acrylates/ C10-30 Alkyl Acrylate Crosspolymer) | 0,1% |
| Water | 91,45% |
| Preservative, Perfume | q.s. |

### Formulierungsbeispiel 23 (nicht erfindungsgemäß)

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| Oxynex® LM | 0,1% |
| Water | 91,4% |
| Preservative, Perfume | q.s. |

### Formulierungsbeispiel 24

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| TEGO® Carbomer 841 SER (Acrylates/ C10-30 Alkyl Acrylate Crosspolymer) | 0,05% |
| Water | 91,45% |
| Preservative, Perfume | q.s. |

### Formulierungsbeispiel 25

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| TEGO® Carbomer 750 HD (Acrylates/ C10-30 Alkyl Acrylate Crosspolymer) | 0,05% |
| Water | 91,45% |
| Preservative, Perfume | q.s. |

### Formulierungsbeispiel 26

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| TEGO® Carbomer 341 ER (Acrylates/ C10-30 Alkyl Acrylate Crosspolymer) | 0,05% |
| Water | 91,45% |
| Preservative, Perfume | q.s. |

### Formulierungsbeispiel 27

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| TEGO® Carbomer 141 (Carbomer) | 0,05% |
| Water | 91,45% |
| Preservative, Perfume | q.s. |

### Formulierungsbeispiel 28

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| Carrageen | 0,05% |
| Water | 91,45% |
| Preservative, Perfume | q.s. |

### Formulierungsbeispiel 29

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| Xantham Gum | 0,1% |
| Water | 91,45% |
| Preservative, Perfume | q.s. |

### Formulierungsbeispiel 30

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| Xantham Gum | 0,05% |
| Carrageen | 0,05% |
| Water | 91,45% |
| Preservative, Perfume | q.s. |

### Formulierungsbeispiel 31

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel3 | 1,0% |
| Hydroxyethylcellulose | 0,1% |
| Propyl Gallate | 0,1% |
| Water | 90,8% |
| Preservative, Perfume | q.s. |

### Formulierungsbeispiel 32

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel3 | 1,0% |
| Hydroxyethylcellulose | 0,1% |
| Propyl Gallate | 0,1% |
| TEGIN® M (Glyceryl Stearate) | 2,0 % |
| Water | 88,8% |
| Preservative, Perfume | q.s. |

### Formulierungsbeispiel 33

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel3 | 1,0% |
| TEGO® Carbomer 341 ER (Acrylates/ C10-30 Alkyl Acrylate Crosspolymer) | 0,1% |
| Water | 91,45% |
| Preservative, Perfume | q.s. |

### Formulierungsbeispiel 34 (nicht erfindungsgemäß)

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| Oxynex® LM | 0,1% |
| Water | 91,4% |
| Preservative, Perfume | q.s. |

### Formulierungsbeispiel 35

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| TEGO® Carbomer 841 SER (Acrylates/ C10-30 Alkyl Acrylate Crosspolymer) | 0,05% |
| Water | 91,45% |
| Preservative, Perfume | q.s. |

### Formulierungsbeispiel 36

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| TEGO® Carbomer 750 HD (Acrylates/ C10-30 Alkyl Acrylate Crosspolymer) | 0,05% |
| Water | 91,45% |
| Preservative, Perfume | q.s. |

### Formulierungsbeispiel 37

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| TEGO® Carbomer 341 ER (Acrylates/ C10-30 Alkyl Acrylate Crosspolymer) | 0,05% |
| Water | 91,45% |
| Preservative, Perfume | q.s. |

### Formulierungsbeispiel 38

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| TEGO® Carbomer 141 (Carbomer) | 0,05% |
| Water | 91,45% |
| Preservative, Perfume | q.s. |

### Formulierungsbeispiel 39

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| Carrageen | 0,05% |
| Water | 91,45% |
| Preservative, Perfume | q.s. |

### Formulierungsbeispiel 40

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| Xantham Gum | 0,1% |
| Water | 91,45% |
| Preservative, Perfume | q.s. |

### Formulierungsbeispiel 41

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| Xantham Gum | 0,05% |
| Carrageen | 0,05% |
| Water | 91,45% |
| Preservative, Perfume | q.s. |

### Formulierungsbeispiel 42

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel4 | 1,0% |
| Hydroxyethylcellulose | 0,1% |
| Propyl Gallate | 0,1% |
| Water | 90,8% |
| Preservative, Perfume | q.s. |

### Formulierungsbeispiel 43

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel4 | 1,0% |
| Hydroxyethylcellulose | 0,1% |
| Propyl Gallate | 0,1% |
| TEGIN® M (Glyceryl Stearate) | 2,0 % |
| Water | 88,8% |
| Preservative, Perfume | q.s. |

### Formulierungsbeispiel 44

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel4 | 1,0% |
| TEGO® Carbomer 341 ER (Acrylates/ C10-30 Alkyl Acrylate Crosspolymer) | 0,1% |
| Water | 91,45% |
| Preservative, Perfume | q.s. |

### Formulierungsbeispiel 45 (nicht erfindungsgemäß)

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| Oxynex® LM | 0,1% |
| Water | 91,4% |
| Preservative, Perfume | q.s. |

### Formulierungsbeispiel 46

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| TEGO® Carbomer 841 SER (Acrylates/ C10-30 Alkyl Acrylate Crosspolymer) | 0,05% |
| Water | 91,45% |
| Preservative, Perfume | q.s. |

### Formulierungsbeispiel 47

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| TEGO® Carbomer 750 HD (Acrylates/ C10-30 Alkyl Acrylate Crosspolymer) | 0,05% |
| Water | 91,45% |
| Preservative, Perfume | q.s. |

### Formulierungsbeispiel 48

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| TEGO® Carbomer 341 ER (Acrylates/ C10-30 Alkyl Acrylate Crosspolymer) | 0,05% |
| Water | 91,45% |
| Preservative, Perfume. | q.s. |

### Formulierungsbeispiel 49

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| TEGO® Carbomer 141 (Carbomer) | 0,05% |
| Water | 91,45% |
| Preservative, Perfume | q.s. |

### Formulierungsbeispiel 50

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| Carrageen | 0,05% |
| Water | 91,45% |
| Preservative, Perfume | q.s. |

### Formulierungsbeispiel 51

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| Xantham Gum | 0,1% |
| Water | 91,45% |
| Preservative, Perfume | q.s. |

### Formulierungsbeispiel 52

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| Xantham Gum | 0,05% |
| Carrageen | 0,05% |
| Water | 91,45% |
| Preservative, Perfume | q.s. |

### Formulierungsbeispiel 53

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel5 | 1,0% |
| Hydroxyethylcellulose | 0,1% |
| Propyl Gallate | 0,1% |
| Water | 90,8% |
| Preservative, Perfume | q.s. |

### Formulierungsbeispiel 54

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 1,0% |
| Synthesebeispiel5 | 1,0% |
| Hydroxyethylcellulose | 0,1% |
| Propyl Gallate | 0,1% |
| TEGIN® M (Glyceryl Stearate) | 2,0 % |
| Water | 88,8% |
| Preservative, Perfume | q.s. |

### Formulierungsbeispiel 55

| | |
|---|---|
| TEGO®Alkanol 1618, Evonik Industries AG (INCI: Cetearyl Alcohol) | 7,0% |
| TEGINACID® C, Evonik Industries AG (INCI: Ceteareth-25) | 0,5% |
| Synthesebeispiel5 | 1,0% |
| TEGO® Carbomer 341 ER (Acrylates/ C10-30 Alkyl Acrylate Crosspolymer) | 0,1% |
| Water | 91,45% |
| Preservative, Perfume | q.s. |

### Formulierungsbeispiel 67 bis 70:

| | 67 | 68 | 69 | 70 |
|---|---|---|---|---|
| Aculyn 46N Polymer; DOW (INCI: PEG-150/stearyl alcohol/SDMI copolymer; 19% ig) | 15.79 | 15.79 | 15.79 | 15.79 |
| Erfindungsgemäßes Beispiel 1 | 1.5 | 1.5 | 1.5 | 2.0 |
| Oleyl Alcohol (85%ig) | | 0.50 | | |
| TEGOSOFT® M, Evonik Nutrition & Care GmbH (INCI:Isopropyl Myristate) | 0.50 | 0.50 | 0.50 | 0.50 |
| TEGO® Pearl N 100; Evonik Nutrition & Care GmbH (INCI: Glycol Distearate; Steareth-4) | 2.00 | 2.00 | | |
| ABIL® ME 45; Evonik Nutrition & Care GmbH (INCI Silicone Quaternium-22; Polyglyceryl-3 Caprate; Dipropylene Glycol; Cocamidopropyl Betaine) | | 1.70 | | |
| Perfume | 0.30 | 0.30 | 0.30 | 0.30 |
| Preservative | q.s. | q.s. | q.s. | q.s. |
| Water | 79.91 | 77.71 | 81.97 | 81.41 |

### Formulierungsbeispiel 71 und 72:

| | 71 | 72 |
|---|---|---|
| Aqupec HU C2002; Sumitomo Seika/Presperse (INCI: Bis-Stearyl Peg/PPG-8/6 SMDI/Peg-400 Copolymer) | 1.00 | 0.75 |
| Structure XL; AkzoNobel (INCI: Hydroxypropyl Starch Phosphate) | | 0.50 |
| Erfindungsgemäßes Beispiel 1 | 2.00 | 2.00 |
| TEGOSOFT® OER; Evonik Nutrition & Care GmbH (INCI: Oleyl Erucate) | 1.00 | 1.00 |
| ABIL® ME 45; Evonik Nutrition & Care GmbH (INCI Silicone Quaternium-22; Polyglyceryl-3 Caprate; Dipropylene Glycol; Cocamidopropyl Betaine) | 1.70 | 1.70 |
| Perfume | 0.30 | 0.30 |
| Preservative | q.s. | q.s. |
| Water | 94.25 | 93.75 |

## Patentansprüche

1. Formulierung enthaltend
A) mindestens ein flüssiges Esterquat, das bei 1 bar einen Schmelzpunkt von 25 °C oder kleiner aufweist,
B) mindestens einen Polymerverdicker,
**dadurch gekennzeichnet,**
**dass** die Formulierung eine Emulsion, insbesondere eine Öl in Wasser Emulsionen, ist, und
**dass** das flüssige Esterquat
Verbindungen der allgemeinen Formel I) 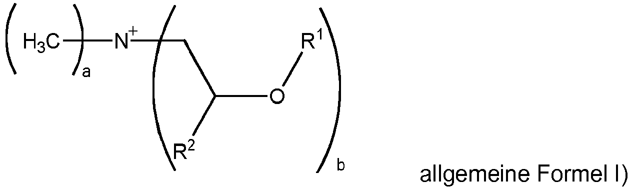 mit R¹ ein Acylrest einer mindestens einfach ungesättigten Fettsäure mit einer Kettenlänge von 18 bis 24 Kohlenstoffatomen oder der Acylrest der Isostearinsäure oder Rizinolsäure,
mit R² ein Alkylrest mit 1 bis 6 Kohlenstoffatomen, bevorzugt Methyl, Ethyl, Propyl, Isopropyl, besonders bevorzugt Methyl,
wobei a = 1 bis 3 und b = 1 bis 3,
mit der Maßgabe, dass a + b =4,
umfasst.

2. Formulierung gemäß Anspruch 1, **dadurch gekennzeichnet, dass** sie zusätzlich mindestens eine Verbindung der allgemeinen Formel Ia) 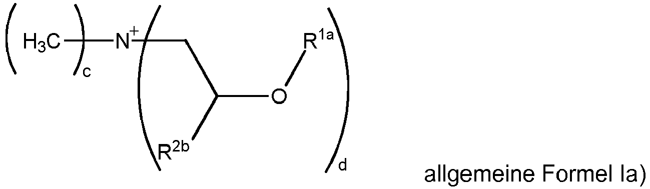
mit R^{1a} ein Acylrest einer anderen Carbonsäure als für R¹ definiert und mit
R^{2b} ein Alkylrest mit 1 bis 6 Kohlenstoffatomen, bevorzugt Methyl, Ethyl, Propyl, Isopropyl, besonders bevorzugt Methyl,
wobei c = 1 bis 3 und d = 1 bis 3,
mit der Maßgabe, dass c + d = 4,
enthält.

3. Formulierung gemäß mindestens einem der vorherigen Ansprüche, **dadurch gekennzeichnet, dass** Komponente A) in einer Menge 0,1 bis 7 Gew.-%, bevorzugt 0,2 bis 5 Gew.-% und insbesondere bevorzugt 0,3 bis 3 Gew.-%, wobei sich die Gew.-% auf die Gesamtformulierung beziehen, enthalten ist.

4. Formulierung gemäß mindestens einem der vorherigen Ansprüche, **dadurch gekennzeichnet, dass** der Polymerverdicker ausgewählt ist aus der Gruppe enthaltend, bevorzugt bestehend aus, natürlichen Verdicker, abgewandelte Naturstoffe, und vollsynthetischen Polymere, Verdicker auf Basis von Silikaten oder modifizierte Tonerden oder deren Derivate und kationische/kationisierte Polymere.

5. Formulierung gemäß mindestens einem der vorherigen Ansprüche, **dadurch gekennzeichnet, dass** der Polymerverdicker ausgewählt ist aus der Gruppe polyacrylatbasierten Verdicker.

6. Formulierung gemäß mindestens einem der vorherigen Ansprüche, **dadurch gekennzeichnet, dass** Komponente B) in einer Menge von 0,005 Gew.-% bis 2 Gew.-%, bevorzugt 0,01 Gew.-% bis 0,5 Gew.-%, besonders bevorzugt 0,05 Gew.-% bis 0,1 Gew.-%, bezogen auf die Gesamtformulierung, enthalten ist.

7. Formulierung gemäß mindestens einem der vorherigen Ansprüche, **dadurch gekennzeichnet, dass** sie zusätzlich enthält
C) mindestens einen Fettalkohol.

8. Formulierung gemäß Anspruch 7, **dadurch gekennzeichnet, dass** der Fettalkohol ausgewählt ist aus der Gruppe enthaltend, bevorzugt bestehend aus, Octanol, Decanol, Laurylalkohol, Isolaurylalkohol, Anteisolaurylalkohol, Myristylalkohol, Isomyristylalkohol, Cetylalkohol, Palmoleylalkohol, Stearylalkohol,Isostearylalkohol, Anteisostearylalkohol, Eicosanol, Petroselinylalkohol, Guerbetalkohol, Arachylalkohol, Gadoleylalkohol, Behenylalkohol, Erucylalkohol, Hectacosanol, Octacosanol, und Melissylalkohol sowie Mischungen davon.

9. Formulierung gemäß Anspruch 7 oder 8, **dadurch gekennzeichnet, dass** Komponente C) in einer Menge von 0,5 bis 20 Gew.-%, bevorzugt 1 bis 10 Gew.-%, insbesondere 2 bis 7 Gew. %, bezogen auf die Gesamtformulierung, enthalten ist.

10. Formulierung gemäß mindestens einem der vorherigen Ansprüche, **dadurch gekennzeichnet, dass** sie zusätzlich enthält
D) mindestens einen Emulgator.

11. Formulierung gemäß mindestens einem der vorherigen Ansprüche, **dadurch gekennzeichnet, dass**
Komponente A) in einer Menge von 0,1 bis 7 Gew.-%, bevorzugt 0,2 bis 5 Gew.-% und insbesondere bevorzugt 0,3 bis 3 Gew.-%,
Komponente B) in einer Menge von 0,005 Gew.-% bis 2 Gew.-%, bevorzugt 0,01 Gew.-% bis 0,5 Gew.-%, besonders bevorzugt 0,05 Gew.-% bis 0,1 Gew.-%, bezogen auf die Gesamtformulierung,
Komponente C) in einer Menge von 0,5 bis 20 Gew.-%, bevorzugt 1 bis 10 Gew.-%, insbesondere 2 bis 7 Gew. %, und
Komponente D) in einer Menge von 0,1 bis 10 Gew.-%, bevorzugt 0,25 bis 5 Gew.-%, insbesondere 0,5 bis 2,5 Gew.-%,
jeweils bezogen auf die Gesamtformulierung, enthalten ist.

## Claims

1. Formulation comprising
A) at least one liquid ester quat having a melting point of 25° or less at 1 bar,
B) at least one polymer thickener,
**characterized in that**,
the formulation is an emulsion, particularly an oil in water emulsion, and
that the liquid ester quat comprises
compounds of the general formula I) 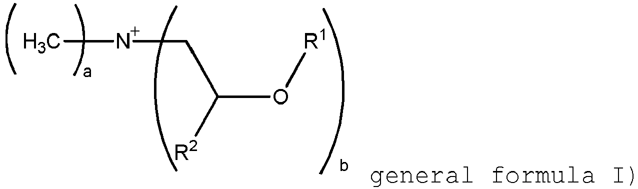
where R¹ is an acyl radical of an at least monounsaturated fatty acid having a chain length of 18 to 24 carbon atoms or the acyl radical of isostearic acid or ricinoleic acid,
where R² is an alkyl radical having 1 to 6 carbon atoms, preferably methyl, ethyl, propyl, isopropyl, particularly preferably methyl,
where a = 1 to 3 and b = 1 to 3,
with the proviso that a + b = 4.

2. Formulation according to Claim 1, **characterized in that** said formulation additionally comprises at least one compound of the general formula Ia) 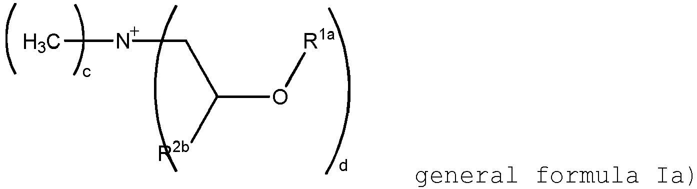
where R^{1a} is an acyl radical of another carboxylic acid as defined for R¹ and
where R^{2b} is an alkyl radical having 1 to 6 carbon atoms, preferably methyl, ethyl, propyl, isopropyl, particularly preferably methyl,
where c = 1 to 3 and d = 1 to 3
with the proviso that c + d = 4.

3. Formulation according to at least one of the preceding claims, **characterized in that** component A) is present in an amount of 0.1 to 7% by weight, preferably 0.2 to 5% by weight and particularly preferably 0.3 to 3% by weight, wherein the percentages by weight refer to the overall formulation.

4. Formulation according to at least one of the preceding claims, **characterized in that** the polymer thickener is selected from the group comprising, preferably consisting of, natural thickeners, modified natural products, and fully synthetic polymers, thickeners based on silicates or modified aluminas or derivatives thereof and cationic/cationized polymers.

5. Formulation according to at least one of the preceding claims, **characterized in that** the polymer thickener is selected from the group of polyacrylatebased thickeners.

6. Formulation according to at least one of the preceding claims, **characterized in that** component B) is present in an amount of 0.005% by weight to 2% by weight, preferably 0.01% by weight to 0.5% by weight, particularly preferably 0.05% by weight to 0.1% by weight, based on the overall formulation.

7. Formulation according to at least one of the preceding claims, **characterized in that** said formulation additionally comprises
C) at least one fatty alcohol.

8. Formulation according to Claim 7, **characterized in that** the fatty alcohol is selected from the group comprising, preferably consisting of, octanol, decanol, lauryl alcohol, isolauryl alcohol, anteisolauryl alcohol, myristyl alcohol, isomyristyl alcohol, cetyl alcohol, palmoleyl alcohol, stearyl alcohol, isostearyl alcohol, anteisostearyl alcohol, eicosanol, petroselinyl alcohol, Guerbet alcohol, arachyl alcohol, gadoleyl alcohol, behenyl alcohol, erucyl alcohol, hectacosanol, octacosanol, and melissyl alcohol and mixtures thereof.

9. Formulation according to Claim 7 or 8, **characterized in that** component C) is present in an amount of 0.5 to 20% by weight, preferably 1 to 10% by weight, particularly 2 to 7% by weight, based on the overall formulation.

10. Formulation according to at least one of the preceding claims, **characterized in that** said formulation additionally comprises
D) at least one emulsifier.

11. Formulation according to at least one of the preceding claims, **characterized in that**
component A) is present in an amount of 0.1 to 7% by weight, preferably 0.2 to 5% by weight and particularly preferably 0.3 to 3% by weight,
component B) is present in an amount of 0.005% by weight to 2% by weight, preferably 0.01% by weight to 0.5% by weight, particularly preferably 0.05% by weight to 0.1% by weight, based on the overall formulation,
component C) is present in an amount of 0.5 to 20% by weight, preferably 1 to 10% by weight, particularly 2 to 7% by weight, and
component D) is present in an amount of 0.1 to 10% by weight, preferably 0.25 to 5% by weight, particularly 0.5 to 2.5% by weight,
based in each case on the overall formulation.

## Revendications

1. Formulation contenant
A) au moins un esterquat liquide, qui présente un point de fusion à 1 bar de 25 °C ou moins,
B) au moins un épaississant de polymère,
**caractérisée en ce que** la formulation est une émulsion, en particulier une émulsion huile-dans-eau, et
**en ce que** l'esterquat liquide comprend des composés de formule générale I) 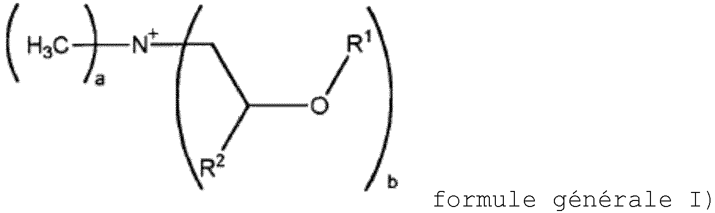
avec R¹ étant un radical acyle d'un acide gras au moins monoinsaturé doté d'une longueur de chaîne de 18 à 24 atomes de carbone ou le radical acyle de l'acide isostéarique ou de l'acide ricinoléïque,
avec R² étant un radical alkyle comportant 1 à 6 atomes de carbone, préférablement méthyle, éthyle, propyle, isopropyle, particulièrement préférablement méthyle,
a = 1 à 3 et b = 1 à 3,
à condition que a + b = 4.

2. Formulation selon la revendication 1, **caractérisée en ce qu'**elle contient de plus au moins un composé de formule générale Ia) 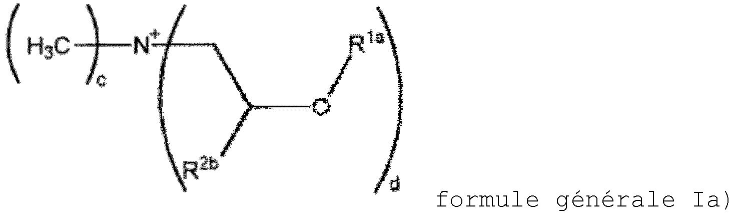
avec R^{1a} étant un radical acyle d'un autre acide carboxylique que défini pour R¹ et
avec R^{2b} étant un radical alkyle comportant 1 à 6 atomes de carbone, préférablement méthyle, éthyle, propyle, isopropyle, particulièrement préférablement méthyle,
c = 1 à 3 et d = 1 à 3,
à condition que c + d = 4.

3. Formulation selon au moins l'une des revendications précédentes, **caractérisée en ce que** le composant A) est contenu en une quantité de 0,1 à 7 % en poids, préférablement 0,2 à 5 % en poids et en particulier préférablement 0,3 à 3 % en poids, les % en poids se rapportant à la formulation totale.

4. Formulation selon au moins l'une des revendications précédentes, **caractérisée en ce que** l'épaississant de polymère est choisi dans le groupe contenant, préférablement constitué par, des épaississants naturels, des matières naturelles modifiées, et des polymères totalement synthétiques, des épaississants à base de silicates ou d'argiles modifiées ou leurs dérivés et des polymères cationiques/cationisés.

5. Formulation selon au moins l'une des revendications précédentes, **caractérisée en ce que** l'épaississant de polymère est choisi dans le groupe des épaississants à base de polyacrylate.

6. Formulation selon au moins l'une des revendications précédentes, **caractérisée en ce que** le composant B) est contenu en une quantité de 0,005 % en poids à 2 % en poids, préférablement 0,01 % en poids à 0,5 % en poids, particulièrement préférablement 0,05 % en poids à 0,1 % en poids, par rapport à la formulation totale.

7. Formulation selon au moins l'une des revendications précédentes, **caractérisée en ce qu'**elle contient de plus
C) au moins un alcool gras.

8. Formulation selon la revendication 7, **caractérisée en ce que** l'alcool gras est choisi dans le groupe contenant, préférablement constitué par, l'octanol, le décanol, l'alcool laurique, l'alcool isolaurique, l'alcool antéisolaurique, l'alcool myristique, l'alcool isomyristique, l'alcool cétylique, l'alcool palmoléïque, l'alcool stéarylique, l'alcool isostéarylique, l'alcool antéisostéarylique, l'éicosanol, l'alcool pétrosélinique, l'alcool de Guerbet, l'alcool arachylique, l'alcool gadoléylique, l'alcool béhénylique, l'alcool érucique, l'hectacosanol, l'octacosanol, et l'alcool mélissylique ainsi que des mélanges correspondants.

9. Formulation selon la revendication 7 ou 8, **caractérisée en ce que** le composant C) est contenu en une quantité de 0,5 à 20 % en poids, préférablement 1 à 10 % en poids, en particulier 2 à 7 % en poids, par rapport à la formulation totale.

10. Formulation selon au moins l'une des revendications précédentes, **caractérisée en ce qu'**elle contient de plus
D) au moins un émulsifiant.

11. Formulation selon au moins l'une des revendications précédentes, **caractérisée en ce que** le composant A) est contenu en une quantité de 0,1 à 7 % en poids, préférablement 0,2 à 5 % en poids et en particulier préférablement 0,3 à 3 % en poids,
le composant B) est contenu en une quantité de 0,005 % en poids à 2 % en poids, préférablement 0,01 % en poids à 0,5 % en poids, particulièrement préférablement 0,05 % en poids à 0,1 % en poids, par rapport à la formulation totale,
le composant C) est contenu en une quantité de 0,5 à 20 % en poids, préférablement 1 à 10 % en poids, en particulier 2 à 7 % en poids, et
le composant D) est contenu en une quantité de 0,1 à 10 % en poids, préférablement 0,25 à 5 % en poids, en particulier 0,5 à 2,5 % en poids,
à chaque fois par rapport à la formulation totale.
